# EUROPEAN PATENT SPECIFICATION

(11) **EP 0 385 335 B1**
(45) Date of publication and mention of the grant of the patent: **03.05.1995**
(21) Application number: 90103682.2
(22) Date of filing: 26.02.1990
(51) Int. Cl.: A61K 47/06, A61K 31/12, A23L 1/303

(54) **Stabilized fat-soluble vitamin compositions**
Stabilisierte fettlösliche Vitaminzusammensetzungen
Compositions stabilisées de vitamines liposolubles

(30) Priority: 28.02.1989 JP 45358/89; 13.02.1990 JP 29778/90
(43) Date of publication of application: 05.09.1990
(73) Proprietor: NISSHIN FLOUR MILLING CO., LTD., Chuo-ku, Tokyo 103 (JP)
(72) Inventor: Matsuda, Yoshihisa, Kyoto-shi, Kyoto-fu (JP); Teraoka, Reiko, Neyagawa-shi, Osaka-fu (JP); Tanaka, Yukio, Kamifukuoka-shi, Saitama-ken (JP); Abe, Hisato, Iruma-gun, Saitama-ken (JP)
(74) Representative: Türk, Gille, Hrabal, Leifert

(56) References cited:
- EP-A- 0 129 003
- CH-A- 375 596
- GB-A- 1 375 436
- D3: Römpps Chemie-Lexikon, 8th ed., 1979, 609 - 610

## Description

This invention relates to stabilized fat-soluble vitamin K compositions. The compositions of the invention have a wide range of applications such as medicines, veterinary medicines, food additives, feed additives, nutrient supplements or the like.

Fat-soluble vitamins such as vitamin K are known to be unstable to light, etc. Thus various methods have been attempted to stabilize those fat-soluble vitamins in formulating pharmaceutical preparations.

For the stabilization of vitamin K₁ (phylloquinone) and vitamin K₂ (menaquinone) having hemostasis-enhancing action (called antihemorrhagic vitamins) which are known to be very unstable to light, attempts have been made wherein vitamin K dissolved in an oil and fat is encapsulated into a colored capsule for light shielding and wherein a variety of stabilizers are incorporated into vitamin K. Japanese Patent Publication No. 4062/1967 discloses a method for the stabilization of menadione (vitamin K₃) or its sodium bisulfite solution using as a stabilizer one or more of caffeine, theobromine, theophylline or their soluble salts.

However the above attempts have been accomplished with unsatisfactory results.

Thus the present invention results from efforts to develop a new stabilizer for fat-soluble vitamin K.

According to the present invention, there is provided a stabilized vitamin K composition which comprises at least 0.017% by weight of vitamin K based on the total weight of the composition and at least 0.01% by weight, based on the weight of the vitamin, of stabilizer selected from the group consisting of β-carotene, γ-carotene, lycopene and canthaxanthin.

Optionally, the compositions of the invention may further comprise oil components and other conventional additives.

One embodiment of the composition includes a solubilized solution which comprises additionally a solubilizing agent and water, optionally together with an oil component and other conventional additives.

Another embodiment of the composition includes an emulsion which comprises additionally an emulsifying agent and water, optionally together with an oil component and other conventional additives.

The fat-soluble vitamin K which is stabilized includes for example vitamin K₁, vitamin K₂, vitamin K₃, vitamin K₄, vitamin K₅, vitamin K₆ and vitamin K₇. Those fat-soluble vitamins, either alone or in combination, can be stabilized with the stabilizer.

The stabilizers which are used in the invention are carotenoids, i.e. β-carotene, γ-carotene, lycopene and canthaxanthin. Those carotenoids may be used either alone or in combination.

These carotenoids are added in an amount effective as the stabilizer for fat-soluble vitamins. Usually, they are added in an amount of at least 0.01% by weight based on the fat-soluble vitamin K. The maximum amount of the carotenoids added is not limited, but preferably is the upper limit of the solubility of the carotenoids in the resulting composition.

The compositions of the invention can be prepared by any methods which allow fat-soluble vitamin K and the carotenoids to be uniformly mixed, for example, by mixing them in an oil component, an organic solvent or the like. Alternatively, both fat-soluble vitamin K and the carotenoids which have been heat-melted may be mixed by stirring.

The oil components which are employed in the invention can include animal oils, vegetable oils, hydrocarbon oils and synthetic glyceride oils, examples of which include various fish oils such as sardin oil, saury oil, squid oil and herring oil; various vegetable oils such as soybean oil, rape oil, corn oil, corn germ oil, cottonseed oil, olive oil, sunflower oil and sesame oil; hydrocarbon oils such as n-hexane, fluid paraffin, squalane, Squalene-EX and Synthelane 30 (both are registered Trade Marks) manufactured by Nikko Chemical Co., Ltd.; and synthetic glycerides such as a medium-chain fatty acid triglyceride called MCT. The organic solvents used include known solvents.

The stabilized fat-soluble vitamin K compositions of the invention can be prepared by adding a stabilizing amount of the specified carotenoids to the oil component in which fat-soluble vitamin K has been dissolved and dissolving the mixture or by adding a desired amount of fat-soluble vitamin K to the oil component in which the carotenoids are dissolved and dissolving the mixture or by mixing three components simultaneously and dissolving the mixture. In this case any methods may be used which can prepare a uniform mixture. If necessary, pharmaceutically acceptable additives may be further added to the stabilized fat-soluble vitamin K compositions.

The emulsifying agents which are employed in the preparation of the stabilized composition in the emulsion form include phospholipids such as yolk phospholipid, soybean phospholipid and modified phospholipids; glycerin fatty acid esters; polyglycerin fatty acid esters such as decaglycerin fatty acid esters; sorbitan fatty acid esters; polyethylene glycol fatty acid esters; polyoxyethylene sorbitan fatty acid esters; polyoxyethylene alkyl ethers; polyoxyethylene castor oil; polyoxyethylene hardened castor oil; polyoxyethylene sorbit fatty acid esters; and polyoxyethylene glycerin fatty acid esters. Phospholipids are preferred.

In the preparation of the stabilized composition in the form of the solubilized solution, the above emulsifying agents can be used as the solubilizing agents depending on the intended purpose.

The emulsion and the solubilized solution can be prepared by emulsifying or solubilizing the stabilized fat-soluble vitamin K composition, for example using any stirring means.

The stabilized fat-soluble vitamin K compositions of the present invention can be used in the suitable preparations. The preparations include liquid preparations such as injections, syrups; solid preparations such as tablets, granules, powders; semi-solid preparations such as creams, ointments; and capsules such as soft and hard capsules.

In forming the above preparations, pharmaceutically acceptable additives can be used, which include other stabilizers than the specified carotenoids; fillers such as lactose, starch, crystalline cellulose, synthetic aluminum silicate; coloring agents such as tar dyes, titanium oxide, yellow iron oxide and red iron oxide; sweetening agents such as white sugar, sodium saccharin, D-xylose, sorbit and mannit; and flavoring agents such as aliphatic alcohols (e.g. peppermint oil), aromatic alcohols and aromatic aldehydes.

The stabilized fat-soluble vitamin K compositions of the invention have high stability to light and do not exhibit reduction in activity even after a long storage. They have a wide range of applications such as medicines, veterinary medicines, food additives, feed additives and nutrient supplements.

The invention is further illustrated by the following examples.

### Example 1

25 mg of vitamin K₂ were added to 23 g of rape oil to prepare Control Sample I. To the above control sample was further added β-carotene in an amount of 0.125 mg, 0.25 mg, 1.25 mg and 2.5 mg to prepare Samples A, B, C and D, respectively.

Each of these samples was irradiated with a white fluorescent lamp at a distance of 30 cm from the light source to the surface of the sample under an illuminance of 2000 ℓm/m² (luces) over a period of 8 hours.

An aliquot of the sample was removed at an interval of one hour during the irradiation and measured for percentage (%) of vitamin K₂ retained in the sample by high performance liquid chromatography.

The results are shown in Table 1.

**Table 1**

| Irradiation time (h) | Sample | | | | |
|---|---|---|---|---|---|
| | A | B | C | D | Control Sample I |
| 1 | 95.47 | 94.80 | 98.17 | 97.62 | 87.39 |
| 2 | 91.53 | 89.68 | 97.22 | 96.26 | 77.76 |
| 3 | 85.22 | 85.95 | 94.13 | 95.31 | 69.11 |
| 4 | 79.79 | 82.69 | 92.80 | 93.83 | 61.93 |
| 5 | 75.10 | 79.42 | 90.64 | 93.60 | 55.50 |
| 6 | 71.17 | 76.34 | 88.75 | 92.93 | 50.58 |
| 7 | 67.70 | 73.25 | 86.81 | 90.84 | 46.04 |
| 8 | 64.34 | 70.21 | 83.47 | 91.03 | 41.68 |

### Example 2

25 mg of vitamin K₂ and 25 mg of vitamin K₁ were independently added to 25 ml of n-hexane to prepare Control Samples II and III, respectively. To each of the above control samples were further added 1.5 mg of β-carotene to prepare Samples E and F, respectively.

Each of these samples was irradiated in the same way as in Example 1 with a white fluorescent lamp under an illuminance of 2000 ℓm/m² (luces) over a period of 8 hours.

An aliquot of the sample was removed at an interval of 1 hour during the irradiation and measured for percentage (%) of vitamin K₂ and vitamin K₁ respectively retained in the sample by high performance liquid chromatography.

The results are shown in Table 2.

**Table 2**

| Irradiation time (h) | Sample | | | |
|---|---|---|---|---|
| | E | Control Sample II | F | Control Sample III |
| 1 | 96.54 | 88.38 | 96.25 | 94.24 |
| 2 | 94.78 | 80.35 | 94.94 | 88.98 |
| 3 | 92.15 | 72.63 | 96.20 | 82.56 |
| 4 | 91.36 | 66.02 | 94.98 | 77.60 |
| 5 | 89.54 | 62.20 | 92.40 | 72.75 |
| 6 | 88.80 | 58.86 | 90.80 | 68.82 |
| 7 | 88.49 | 54.83 | 91.17 | 64.08 |
| 8 | 86.85 | 51.33 | 90.06 | 61.70 |

### Example 3

25 mg of vitamin K₂ were added to 25 ml of n-hexane to prepare Control Sample IV. 1.58 mg of canthaxanthin and 1.5 mg of lycopen, respectively were added to the above control sample to prepare Samples G and H, respectively.

Each of these samples was irradiated in the same way as in Example 1 with a white fluorescent lamp under an illuminance of 2000 ℓm/m² (luces) over a period of 8 hours.

An aliquot of the sample was removed at an interval of 1 hour during the irradiation and measured for percentage (%) of vitamin K₂ retained in the sample by high performance liquid chromatography.

The results are shown in Table 3.

**Table 3**

| Irradiation time (h) | Sample | | |
|---|---|---|---|
| | G | H | Control Sample IV |
| 1 | 98.04 | 97.44 | 88.38 |
| 2 | 96.55 | 95.01 | 80.35 |
| 3 | 94.86 | 93.02 | 72.63 |
| 4 | 92.18 | 91.62 | 66.02 |
| 5 | 90.99 | 90.43 | 62.20 |
| 6 | 89.71 | 89.43 | 58.86 |
| 7 | 89.11 | 89.33 | 54.83 |
| 8 | 88.64 | 88.31 | 51.33 |

The above results indicate that canthaxanthin and lycopen are effective as a stabilizer to prevent light degradation of vitamin K₂.

### Example 4

25 mg of vitamin K₂ were added to 25 ml of n-hexane to prepare Control Sample V. 0.3 mg of β-carotene, 0.316 mg of canthaxanthin, 0.3 mg of lycopen and 0.3 mg of γ-carotene, respectively were added to the above control sample to prepare Samples I, J, K and L, respectively.

Each of these samples was irradiated in the same way as in Example 1 with a white fluorescent lamp under an illuminance of 2000 ℓm/m² (luces) over a period of 8 hours.

An aliquot of the sample was removed at an interval of 1 hour during the irradiation and measured for percentage (%) of vitamin K₂ retained in the sample by high performance liquid chromatography.

The results are shown in Table 4.

**Table 4**

| Irradiation time (h) | Sample | | | | |
|---|---|---|---|---|---|
| | I | J | K | L | Control Sample V |
| 1 | 95.22 | 97.40 | 99.90 | 97.12 | 88.38 |
| 2 | 91.94 | 93.26 | 94.98 | 92.40 | 80.35 |
| 3 | 90.72 | 91.19 | 92.73 | 90.20 | 72.63 |
| 4 | 86.86 | 88.48 | 90.19 | 87.58 | 66.02 |
| 5 | 84.77 | 85.40 | 87.65 | 85.71 | 62.20 |
| 6 | 81.86 | 85.39 | 85.19 | 83.86 | 58.86 |
| 7 | 79.62 | 81.79 | 84.86 | 82.59 | 54.83 |
| 8 | 78.36 | 79.27 | 79.44 | 80.67 | 51.33 |

### Example 5

Vitamin K₁, β-carotene and rape oil were mixed in such a proportion that one capsule contained 5 mg of vitamin K₁, 0.006 mg of β-carotene and 245 mg of rape oil. The resulting liquid mixture was encapsulated by a conventional method with gelatin coat to form soft capsules (Sample M). Separately, soft capsules (Control Sample VI) were prepared from the same liquid mixture as above but containing no β-carotene.

These samples were irradiated with a white fluorescent lamp under an illuminance of 1000 ℓm/m² (luces) and measured for percentage of vitamin K₁ retained in the sample.

The results are shown in Table 5.

**Table 5**

| Irradiation time (h) | Sample | |
|---|---|---|
| | M | Control Sample VI |
| 6 | 101.8 | 97.4 |
| 30 | 100.8 | 82.2 |
| 120 | 96.4 | 27.4 |

### Example 6

5.0 g of vitamin K₁ and 0.126 g of β-carotene were dissolved in 50 ml of a mixed solvent (ethanol:chloroform = 1:1) and to the solution were added 138 g of lactose, 101 g of Avicel® and 8 g of PVP. The resulting mixture was kneaded, granulated, dried and graded to prepare granules as Sample N.

Separately, the same granules as above but containing no β-carotene (Control Sample VII) were prepared in the same way as above.

These samples were irradiated with a white fluorescent lamp under an illuminance of 1000 ℓm/m² (luces) and measured for percentage (%) of vitamin K₁ retained in the sample.

The results are shown in Table 6.

**Table 6**

| Irradiation time (h) | Sample | |
|---|---|---|
| | N | Control Sample VII |
| 24 | 90.3 | 82.8 |
| 72 | 87.8 | 75.0 |
| 120 | 80.8 | 71.5 |

### Example 7

To 50 mg of vitamin K₁, 1 mg of β-carotene and 2000 mg of polyoxyethylene hardened castor oil (HCO-60 manufactured by Nikko Chemical Co., Ltd.) were added 300 ml of distilled water. The mixture was warmed with stirring to prepare a solubilized solution as Sample O.

Separately, the same solubilized solution as above but containing no β-carotene was prepared as Control Sample VIII.

The same procedure as mentioned above was repeated but substitution of vitamin K₂ for vitamin K₁ to prepare Sample P. The same solubilized solution as above but containing no β-carotene was prepared as Control Sample IX.

These samples were respectively placed in a transparent beaker and irradiated with a white fluorescent lamp under an illuminance of 2000 ℓm/m² (luces) on the surface of the sample over a period of 6 hours.

An aliquot of the sample was removed at intervals shown in the table below during the irradiation and measured for percentage (%) of vitamin K₁ and vitamin K₂ retained in the sample.

The results are shown in Table 7.

**Table 7**

| Irradiation time (h) | Sample | | | |
|---|---|---|---|---|
| | O | Control Sample VIII | P | Control Sample IX |
| 0.5 | 96.8 | 90.5 | 98.3 | 92.8 |
| 1 | 93.7 | 84.7 | 93.9 | 85.5 |
| 2 | 90.7 | 76.9 | 86.8 | 69.2 |
| 3 | 86.6 | 70.4 | 83.7 | 67.4 |
| 4 | 82.5 | 63.6 | 80.9 | 63.7 |
| 5 | 78.9 | 58.5 | 81.0 | 59.2 |
| 6 | 76.8 | 57.6 | 75.7 | 53.8 |

### Example 8

To distilled water were added 50 mg of vitamin K₂, 10.0 g of soybean oil, 1.2 g of soybean lecithin, 2.5 g of glycerin and 1 mg of β-carotene to a total volume of 300 ml. The mixture was emulsified to prepare a vitamin K₂-containing emulsion as Sample Q.

Separately, the same emulsion as above but containing no β-carotene was prepared as Control Sample X.

The same procedure as mentioned above was repeated but substitution of vitamin K₁ for vitamin K₂ to prepare Sample R. The same emulsion as above but containing no β-carotene was prepared as Control Sample XI.

These samples were irradiated with a white fluorescent lamp under an illuminance of 2000 ℓm/m² (luces) and measured for percentage (%) of vitamin K₂ and vitamin K₁ retained in the sample.

The results are shown in Table 8.

**Table 8**

| Irradiation time (h) | Sample | | | |
|---|---|---|---|---|
| | Q | Control Sample X | R | Control Sample XI |
| 0.5 | 99.8 | 90.2 | 99.3 | 93.7 |
| 1 | 98.7 | 86.2 | 97.9 | 90.3 |
| 2 | 97.4 | 79.1 | 96.5 | 84.6 |
| 3 | 94.6 | 72.3 | 94.8 | 78.9 |
| 4 | 92.5 | 71.8 | 94.4 | 74.5 |
| 5 | 92.5 | 68.1 | 91.5 | 69.6 |
| 6 | 86.9 | 62.4 | 90.7 | 65.7 |

The above results indicate that the presence of β-carotene in the vitamin K₂ or K₁-containing emulsion provides remarkably improved light stability of vitamin K₂ or K₁.

## Claims

1. A stabilized vitamin K composition which comprises at least 0.017% by weight of vitamin K based on the total weight of the composition and at least 0.01% by weight, based on the weight of the vitamin, of stabilizer selected from the group consisting of β-carotene, γ-carotene, lycopene and canthaxanthin.

2. A composition of claim 1 which further comprises a pharmaceutically acceptable oil carrier.

3. A composition of claim 1 therein it is formulated into a solubilized solution.

4. A composition of claim 1 wherein it is formulated into an emulsion.

5. A composition of claim 1 wherein it is in the form of a semi-solid.

6. A composition of claim 1 wherein it is in the form of a solid.

7. A composition of claim 1 wherein the vitamin K is vitamin K₁, vitamin K₂, vitamin K₃, vitamin K₄, vitamin K₅, vitamin K₆ or vitamin K₇.

8. A composition of claim 2 wherein the oil carrier is animal, vegetable, hydrocarbon and synthetic glyceride oils.

9. A pharmaceutical preparation selected from the group consisting of liquid preparations, solid preparations, semi-solid preparations and capsules, which comprises the stabilized vitamin K compositions of claim 1 and pharmaceutically acceptable additives.

10. The use of β-carotene, γ-carotene, lycopene and canthaxanthin as a stabilizer for vitamin K.

## Patentansprüche

1. Stabilisierte Vitamin K-Zusammensetzung, die umfaßt bzw. enthält mindestens 0,017 Gew.-% Vitamin K, bezogen auf das Gesamtgewicht der Zusammensetzung, und mindestens 0,01 Gew.-%, bezogen auf das Gewicht des Vitamins, Stabilisator, ausgewählt aus der Gruppe, die besteht aus β-Carotin, γ-Carotin, Lycopen und Canthaxanthin.

2. Zusammensetzung nach Anspruch 1, die außerdem einen pharmazeutisch akzeptablen Ölträger umfaßt bzw. enthält.

3. Zusammensetzung nach Anspruch 1, die als solubilisierte Lösung formuliert ist.

4. Zusammensetzung nach Anspruch 1, die als Emulsion formuliert ist.

5. Zusammensetzung nach Anspruch 1, die in Form eines halbfesten Präparats vorliegt.

6. Zusammensetzung nach Anspruch 1, die in Form eines Feststoffes vorliegt.

7. Zusammensetzung nach Anspruch 1, die als Vitamin K Vitamin K₁, Vitamin K₂, Vitamin K₃, Vitamin K₄, Vitamin K₅, Vitamin K₆ oder Vitamin K₇ enthält.

8. Zusammensetzung nach Anspruch 2, die als Ölträger tierische, pflanzliche, Kohlenwasserstoff- und synthetische Glycerid-Öle enthält.

9. Pharmazeutisches Präparat, ausgewählt aus der Gruppe der flüssigen Präparate, festen Präparate, halbfesten Präparate und Kapseln, das umfaßt bzw. enthält die stabilisierten Vitamin K-Zusammensetzungen nach Anspruch 1 und pharmazeutisch akzeptable Additive (Zusätze).

10. Verwendung von β-Carotin, γ-Carotin, Lycopen und Canthaxanthin als Stabilisator für Vitamin K.

## Revendications

1. Composition stabilisée de vitamine K qui comprend au moins 0,017% en poids de vitamine K par rapport au poids total de la composition et au moins 0,01% en poids, par rapport au poids de la vitamine, d'un stabilisant choisi dans le groupe formé par le β-carotène, le γ-carotène, le lycopène et la canthaxantine.

2. Composition selon la revendication 1, qui comprend de plus un véhicule huileux acceptable sur le plan pharmaceutique.

3. Composition selon la revendication 1, qui est formulée en une solution solubilisée.

4. Composition selon la revendication 1, qui est formulée en une émulsion.

5. Composition selon la revendication 1, qui est sous forme d'un semi-solide.

6. Composition selon la revendication 1, qui est sous forme d'un solide.

7. Composition selon la revendication 1, dans laquelle la vitamine K est la vitamine K₁, la vitamine K₂, la vitamine K₃, la vitamine K₄, la vitamine K₅, la vitamine K₆ ou la vitamine K₇.

8. Composition selon la revendication 2, dans laquelle le véhicule huileux est une huile animale, végétale, hydrocarbonée et de glycéride synthétique.

9. Préparation pharmaceutique choisie dans le groupe formé par les préparations liquides, les préparations solides, les préparations semi-solides et les gélules, qui comprend les compositions de vitamine K solubilisées selon la revendication 1 et des additifs acceptables sur le plan pharmaceutique.

10. Utilisation de β-carotène, de γ-carotène, de lycopène et de canthaxanthine en tant que stabilisant pour la vitamine K.
